# EUROPÄISCHE PATENTSCHRIFT

(11) **EP 2 909 812 B1**
(45) Veröffentlichungstag und Bekanntmachung des Hinweises auf die Patenterteilung: **23.08.2017**
(21) Anmeldenummer: 13779828.6
(22) Anmeldetag: 21.10.2013
(51) Int. Cl.: G06T 11/00, A61B 6/02

(54) **VERFAHREN UND VORRICHTUNG ZUM ERZEUGEN EINER DREIDIMENSIONALEN ABBILDUNG EINES OBJEKTS**
METHOD AND DEVICE FOR GENERATING A THREE-DIMENSIONAL IMAGE OF AN OBJECT
PROCÉDÉ ET DISPOSITIF POUR GÉNÉRER UNE REPRODUCTION EN TROIS DIMENSIONS D'UN OBJET

(30) Priorität: 22.10.2012 DE 102012219269
(43) Veröffentlichungstag der Anmeldung: 26.08.2015
(73) Patentinhaber: Fraunhofer-Gesellschaft zur Förderung der angewandten Forschung e.V., 80686 München (DE); Charité - Universitätsmedizin Berlin, 10117 Berlin (DE)
(72) Erfinder: KEEVE, Erwin, 14469 Potsdam (DE); KÄSEBERG, Marc, 16359 Biesenthal (DE); STOPP, Fabian, 15366 Neuenhagen (DE); UHLMANN, Eckart, 25368 Kiebitzreihe (DE); ENGEL, Sebastian, 48147 Münster (DE)
(74) Vertreter: Pfenning, Meinig & Partner mbB
(86) Internationale Anmeldenummer: PCT/EP2013/071954
(87) Internationale Veröffentlichungsnummer: WO 2014/064042

(56) Entgegenhaltungen:
- US-A1- 2010 202 583
- US-A1- 2010 296 624

## Beschreibung

Die vorliegende Erfindung betrifft ein Verfahren sowie eine Vorrichtung zum Erzeugen einer dreidimensionalen Abbildung eines Objekts. Ferner betrifft die vorliegende Erfindung ein Computerprogrammprodukt, das eine Befehlsfolge enthält, die eine Vorrichtung zum Erzeugen einer dreidimensionalen Abbildung eines Objekts ansteuert.

Abbildungsvorrichtungen, wie beispielsweise Röntgensysteme, dienen in der Medizin der Untersuchung von Patienten, wobei bei besonderen Ausgestaltungen der jeweiligen Abbildungsvorrichtung aus zweidimensional aufgenommenen Bildern durch ein Rechenverfahren eine dreidimensionale Abbildung rekonstruiert werden kann.

So werden beispielsweise bei der digitalen Volumentomographie (DVT) mit einem Volumentomographen mehrere Bilder durch eine um einen Patienten rotierende Röntgenröhre und einen der Röntgenröhre während einer Rotation gegenüberliegenden Sensor aufgenommen und anschließend zu einer dreidimensionalen Rekonstruktion eines gescannten Gebietes weiterverarbeitet. Die digitale Volumentomographie als medizinisches Bildgebungsverfahren wird weitestgehend zur dreidimensionalen Bildaufnahme des Schädels verwendet. Da der digitale Volumentomograph die Röntgenquelle und den ihr gegenüber liegenden Sensor zumeist horizontal auf einer Kreisbahn bewegt, weist das Verfahren eine deutliche Artefaktbildung bei stark röntgenstrahlenabsorbierenden Materialien, wie beispielsweise Metallimplantaten, Zahnfüllungen oder Zahnspangen auf. Diese Materialien löschen Information über vor- und nachgelagertes Gewebe in zweidimensionalen Bilddaten aus oder schwächen diese zumindest stark ab. Dies führt zu Strukturen im rekonstruierten dreidimensionalen Bild, die nicht mit räumlichen Verteilungen vom zu untersuchendem Gewebe in einer Bildebene übereinstimmen, den sogenannten Metallartefakten.

Aus dem Stand der Technik sind bereits Verfahren und Vorrichtungen bekannt, mittels derer diese Artefakte zur diagnostischen Verwertbarkeit reduziert werden können. Bisherige Lösungsansätze basieren hierzu auf algorithmischen Anpassungen in einer Datenverarbeitung vor, während oder nach der Rekonstruktion der dreidimensionalen Abbildung. Beispielsweise offenbart die Druckschrift DE 103 20 233 A1 eine Berechnung von Artefaktprojektionsbildern auf Basis einer vorläufigen dreidimensionalen Rekonstruktion und darin detektierter metallischer Teile. Diese Artefaktprojektionsbilder werden nachfolgend dazu genutzt, die ursprünglichen zweidimensionalen Projektionsbilder zu verbessern, was schließlich zu einer Reduktion der Metallartefakte in den endgültigen dreidimensionalen Bilddaten führt. Ein genereller Nachteil solcher algorithmischer Lösungen ist jedoch, dass Korrekturverfahren die Auswirkungen eines Informationsverlusts durch eine Absorption der Metallteile zu reduzieren versuchen bzw. versuchen, die verlorenen Daten durch Inter- bzw. Extrapolation wieder herzustellen, wobei die während der Aufnahme ausgelöschten Daten allerdings nicht vollständig rekonstruiert werden können.

Aus der Druckschrift US 2010/0296624 A1 ist ein Computertomograph bekannt, bei dem das abzubildende Objekt auf einer Zylinderfläche umfahren wird. Auch die Druckschrift US 2010/0202583 A1 offenbart einen Computertomographen, bei dem eine Strahlenquelle einen Patienten auf einer Zylinderfläche umfährt.

Der vorliegenden Erfindung liegt damit die Aufgabe zu Grunde, ein Verfahren und eine Vorrichtung zu entwickeln, mit denen die genannten Nachteile vermieden werden, mit denen also eine Reduzierung von Artefakten bei einer dreidimensionalen Abbildung eines Objekts einfach und schnell ohne nachfolgende algorithmische Bearbeitung von aufgenommen Bilddaten erfolgt.

Diese Aufgabe wird erfindungsgemäß gelöst durch ein Verfahren nach Anspruch 1, eine Vorrichtung nach Anspruch 10, sowie ein Computerprogrammprodukt nach Anspruch 14 zum Steuern einer Vorrichtung bzw. Durchführen eines Verfahrens. Vorteilhafte Weiterbildungen werden in den Unteransprüchen beschrieben.

Ein erfindungsgemäßes Verfahren bedient sich zum Erzeugen einer dreidimensionalen Abbildung eines Objekts einer Abbildungsvorrichtung. Die Abbildungsvorrichtung umfasst zum Aufnehmen von Bildern des abzubildenden Objekts aus mehreren Positionen eine bewegliche Strahlenquelle, einen Strahlendetektor und eine Auswerteeinheit. Die Strahlenquelle wird zu einer Bildaufnahme relativ zu dem abzubildenden Objekt auf einer ersten, in einer ersten Ebene liegenden Bahn in einer ersten Bewegung in mehrere Positionen bewegt. In den Positionen wird jeweils mindestens ein Bild aufgenommen, wobei aus den aufgenommenen Bildern durch die Auswerteeinheit die dreidimensionale Abbildung rekonstruiert wird. Zum Reduzieren von einem durch eine Abschattung erzeugten Artefakt in der Abbildung führt die Strahlenquelle jedoch zeitgleich zu der ersten Bewegung eine zweite Bewegung auf einer zumindest abschnittsweise von der ersten Bahn verschiedenen zweiten Bahn relativ zum abzubildenden Objekt durch. Die erste Bewegung und die zweite Bewegung überlagern sich hierbei. Ein Abstand zwischen der Strahlenquelle und dem abzubildenden Objekt ist während des Überlagerns der ersten Bewegung und der zweiten Bewegung, also während der Bewegung der Strahlenquelle, konstant.

Durch die Überlagerung der ersten Bewegung und der zweiten Bewegung, die bewirkt, dass diese gleichzeitig vollführt werden, und das Aufnehmen von Bildern in verschiedenen Positionen werden zusätzliche Projektionsperspektiven generiert. Durch eine Ergänzung der ersten Bewegung um die zweite Bewegung, d. h. eine Überlagerung mit mindestens einer weiteren Bewegung bzw. mindestens einem weiteren Freiheitsgrad, wird eine höhere Bildqualität und eine bessere technische Verwertbarkeit erzielt. Die neuen Projektionsperspektiven enthalten Projektions- bzw. Bildinformationen über das abzubildende Objekt, die durch eine Bildaufnahme entlang der ersten Bahn alleine nicht erfassbar sind. Durch den konstanten Abstand zwischen der Strahlenquelle und dem abzubildenden Objekt ist überdies eine einfache geometrische Verknüpfung gegeben, die die Auswertung erleichtert. Insbesondere enthalten diese zusätzlichen Projektionsperspektiven Informationen, die bei einer herkömmlichen Bildaufnahme durch Metallkomponenten, wie beispielsweise Zahnimplantate oder Zahnfüllungen bei Untersuchungen eines Schädels, ausgelöscht würden. Auf Basis der zusätzlich erhaltenen Informationen ist eine exakte Darstellung des gescannten Objekts möglich, ohne hierfür einen gesonderten Algorithmus zum Reduzieren von Artefakten durchführen zu müssen. Hierdurch wird sowohl eine Geschwindigkeit, mit der das Abbildungsverfahren durchgeführt wird, erhöht, als auch eine Qualität der erhaltenen Bilddaten verbessert. Durch die Bewegung der Strahlenquelle auf einer kombinierten Bahn, die sich aus der ersten Bahn und der zweiten Bahn ergibt, können sowohl die Vorteile der Geometrie der ersten Bahn als auch die der zweiten Bahn ausgenutzt werden. Es werden somit während des Aufnehmens zwei Bewegungen vollführt, die zu unterschiedlichen Aufnahmepositionen und neuen Projektionsperspektiven führen. Die kombinierte Bahn, auf der die überlagerte Bewegung stattfindet, beschreibt hierbei die Bewegung der Strahlenquelle im Raum. Unter einer Bahn soll in diesem Zusammenhang eine Kombination von Raumpunkten verstanden werden, die die Strahlenquelle nacheinander durchläuft bzw. ohne Überlagerung durchlaufen würde. Die kombinierte Bahn ist also gerade die Trajektorie der Bewegung, die sich aus den einzelnen Punkten zusammensetzt, die während des Aufnehmens durchlaufen werden.

Zumindest ein Teil des abzubildenden Objekts, vorzugsweise ein Mittelpunkt des abzubildenden Objekts, kann in einem Achsenschnittpunkt einer ersten Achse, um die die erste Bewegung erfolgt, und einer zweiten Achse, um die die zweite Bewegung erfolgt, liegen. Der Mittelpunkt kann sowohl ein geometrischer Mittelpunkt als auch ein Massenschwerpunkt sein. Somit wird sichergestellt, dass das eigentlich zu untersuchende und interessante Zielgebiet, welches räumlich durch den Mittelpunkt definiert ist, sowohl von der ersten Bewegung als auch von der zweiten Bewegung umlaufen wird, und somit Bildinformationen unter verschiedenen Winkeln über dieses Zielgebiet verfügbar sind. Zugleich wird hiermit eine einfache Weiterverarbeitung der erhaltenen Bilddaten ermöglicht. Vorzugsweise ist ein Abstand zwischen der Strahlenquelle und dem Mittelpunkt und bzw. oder ein Abstand zwischen dem Strahlendetektor und dem Mittelpunkt während der ersten Bewegung und der zweiten Bewegung konstant.

Typischerweise steht die erste Achse senkrecht auf der ersten Ebene und bzw. oder stehen die erste Achse und die zweite Achse senkrecht aufeinander, sodass eine besonders klare geometrische Relation zwischen der ersten Bewegung und der zweiten Bewegung gegeben ist und eine entsprechend vereinfachte Weiterbearbeitung durchgeführt werden kann. Insbesondere kann die erste Achse auch komplett in einer zweiten Ebene der zweiten Bewegung liegen und bzw. oder die zweite Achse komplett in der ersten Ebene liegen. Es kann ferner vorgesehen sein, dass eine Mittelachse von einem von der Strahlenquelle ausgesandten Strahlenbündel durch den Achsenschnittpunkt verläuft, sodass das Gebiet um diesen Achsenschnittpunkt mit genügend hoher Intensität beleuchtet und in entsprechender Qualität abgebildet wird.

Der Abstand zwischen der Strahlenquelle und dem abzubildenden Objekt kann als Abstand zwischen einem geometrischen Mittelpunkt oder einem Masseschwerpunkt der Strahlenquelle und dem geometrischen Mittelpunkt oder dem Masseschwerpunkt des abzubildenden Objekts definiert sein. Es kann aber auch vorgesehen sein, statt des geometrischen Mittelpunkts oder des Masseschwerpunkts des abzubildenden Objekts einen Bezugspunkt auf der Oberfläche für die Bestimmung des Abstands zu nehmen, wobei vorzugsweise ein der Strahlenquelle am nächsten liegender Punkt der Oberfläche als Bezugspunkt dient.

Der Abstand zwischen der Strahlenquelle und dem abzubildenden Objekt und der Abstand zwischen dem Strahlendetektor und dem abzubildenden Objekt sind typischerweise gleich groß, um eine einfache geometrische Verknüpfung zwischen der Strahlenquelle, dem abzubildenden Objekt und dem Strahlendetektor zu haben. Hierdurch wird ein Auswerten vereinfacht. Alternativ kann der Abstand zwischen der Strahlenquelle und dem abzubildenden Objekt, typischerweise dem Mittelpunkt des abzubildenden Objekts, auch kleiner als der Abstand zwischen dem Strahlendetektor und dem abzubildenden Objekt, auch hier wieder typischerweise dem Mittelpunkt des abzubildenden Objekts, sein.

Typischerweise erfolgt die zweite Bewegung zumindest abschnittsweise in einem von zwei durch die erste Ebene definierten Halbräumen. Die erste Ebene unterteilt somit einen das abzubildende Objekt umgebenden Raum in zwei Halbräume, sodass die zweite Bewegung beispielsweise nur in einem der beiden Halbräume, also insbesondere ausschließlich unterhalb bzw. oberhalb oder links bzw. rechts der ersten Ebene erfolgen kann, oder die zweite Bewegung in beiden Halbräumen erfolgt, wobei die erste Ebene zu bestimmten Zeiträumen während der zweiten Bewegung durchlaufen wird. Somit ist, je nach gewünschtem Einsatzgebiet, eine Aufnahme von Bildern in einer Vielzahl von Positionen möglich und die Bewegung individuell einstellbar. Vorzugsweise erfolgt die zweite Bewegung jedoch zumindest abschnittsweise in beiden Halbräumen und bzw. oder die zweite Bewegung ist zumindest abschnittsweise eine periodische Bewegung, besonders vorzugsweise eine sinusförmige oder eine in diskreten Schritten erfolgende Bewegung. Insbesondere kann die zweite Bewegung auch komplett aus einer periodischen Bewegung bestehen. Durch eine Periodizität der jeweiligen Bewegung wird eine Weiterverarbeitung der erhaltenen Daten deutlich vereinfacht. Die zweite Bewegung kann natürlich auch einem frei wählbaren Verlauf folgen. Somit ist eine Einstellung der zweiten Bewegung je nach der Geometrie des abzubildenden Objekts möglich.

Typischerweise ist die erste Bewegung eine Kreisbewegung, eine ellipsenförmige Bewegung oder eine in ihrem Verlauf frei wählbare Bewegung und bzw. oder die zweite Bewegung eine Neigebewegung. Die erste Bewegung, insbesondere die Kreisbewegung, kann vorzugsweise horizontal oder vertikal beispielsweise bei sitzenden oder liegenden Patienten oder Objekten erfolgen. Außerdem kann die erste Bahn eine geschlossene Bahn sein, d.h. eine Bahn, bei der ein Anfangspunkt mit einem Endpunkt zusammenfällt. Dementsprechend kann die erste Bewegung eine geschlossene Bewegung sein, bei der der Anfangspunkt dem Endpunkt entspricht. Hierdurch wird typischerweise durch die erste Bewegung das abzubildende Objekt zumindest einmal vollständig umfahren, sodass Bildinformationen aus rund um das abzubildende Objekt verlaufenden Perspektiven verfügbar sind. Es kann natürlich auch vorgesehen sein, eine offene, also nicht geschlossene Bahn für die erste Bahn zu verwenden bzw. eine offene Bewegung durchzuführen.

Durch eine Neigung der Neigebewegung wird die Perspektive jeweils geändert und zusätzliche Bildinformationen verfügbar gemacht. Typischerweise wird zumindest die Strahlenquelle in der ersten Bewegung auf der ersten Bahn, insbesondere der Kreisbewegung auf einer Kreisbahn, um mindestens 90°, vorzugsweise mind. 180°, besonders vorzugsweise um mind. 360° bewegt. Alternativ oder zusätzlich kann zumindest die Strahlenquelle in der Neigebewegung gegenüber einer Ebene der der ersten Bewegung um max. zwischen 1° und 45°, vorzugsweise max. zwischen 10° und 35°, besonders vorzugsweise max. zwischen 15° und 30° geneigt sein. Durch die angegebenen Winkelbereiche werden Bilder aus einer Vielzahl von Perspektiven ermöglicht, ohne dass die Strahlenquelle zu stark verfahren werden muss.

Die Bahn der ersten Bewegung und die Bahn der zweiten Bewegung können an das zu untersuchende Objekt angepasst sein, um ein optimales Rekonstruktionsvolumen und bzw. oder eine möglichst hohe Ortsauflösung zu erreichen.

Typischerweise wird der Strahlendetektor bei der Bewegung der Strahlenquelle mitbewegt, jedoch ist es auch möglich, den Strahlendetektor ortsfest zu belassen und lediglich die Strahlenquelle zu bewegen. Ebenso ist es auch möglich, dass der Strahlendetektor die erste Bewegung und die zweite Bewegung der Strahlenquelle mitvollführt oder auch nur eine der beiden Bewegungen gemeinsam mit der Strahlenquelle nachvollzieht. Typischerweise wird der Strahlendetektor hierbei nur in der Kreisbewegung geführt, während er die Neigebewegung nicht vollführt, natürlich kann er aber auch nur die Neigebewegung mitmachen und die Kreisbewegung nicht mit vollziehen. Vorzugsweise ist der Strahlendetektor unabhängig von der Strahlenquelle bewegbar.

Es ist außerdem möglich, dass der Neigungswinkel um die Neigungsachse, also um die zweite Achse, variabel, also nicht konstant, während der Rotationsbewegung bzw. Kreisbewegung um die erste Achse, d.h. der Rotationsachse, ist. Hierdurch wird es ebenfalls möglich, an die Geometrie des abzubildenden Objekts angepasste Positionen für eine Bildaufnahme auszuwählen.

Es kann ferner vorgesehen sein, die erste Bewegung und bzw. oder die zweite Bewegung zumindest abschnittsweise automatisiert und bzw. oder zumindest abschnittsweise manuell angetrieben durchzuführen. Die Einstellung der für die Bildaufnahme verwendeten Positionen kann somit vorgegeben und ohne Zutun eines Benutzers angefahren bzw. direkt und unmittelbar nach den Wünschen des Benutzers eingestellt werden. Typischerweise ist eine Geschwindigkeit der ersten Bewegung und bzw. oder eine Geschwindigkeit der zweiten Bewegung konstant oder abschnittsweise variabel. Die Geschwindigkeit der Bewegungen ist vorzugsweise eine Winkelgeschwindigkeit. Dies erlaubt es, das Verfahren schneller durchzuführen, da auch die Winkelgeschwindigkeit an eine Anzahl von in den jeweiligen Positionen aufzunehmenden Bildern angepasst werden kann. Die Winkelgeschwindigkeit der zweiten Bewegung kann zumindest abschnittsweise, vorzugsweise stets höher als die Winkelgeschwindigkeit der ersten Bewegung sein. Besonders vorzugsweise erfolgt bei einem Erhöhen der Winkelgeschwindigkeit der zweiten Bewegung, also einer Beschleunigung, ein Herabsetzen, also ein Verlangsamen, der Winkelgeschwindigkeit der ersten Bewegung, um durch verringerte Beschleunigungskräfte eine hohe Genauigkeit der Aufnahme zu gewährleisten. Umgekehrt kann bei einer Verringerung der Winkelgeschwindigkeit der zweiten Bewegung ein Erhöhen der Winkelgeschwindigkeit der ersten Bewegung erfolgen. Hierdurch erhält man von besonders interessierenden Bereichen viele Aufnahmen unter unterschiedlichen Neigungswinkeln während ein vergleichsweise geringer Winkelbereich der Rotationsbewegung in der gleichen Zeit überstrichen wird.

Die Strahlenquelle wird vorzugsweise auf einer virtuellen Kugeloberfläche bewegt, wobei ein Mittelpunkt der Kugel mit dem Bezugspunkt zusammenfällt, der den objektseitig den Abstand zwischen der Strahlenquelle und dem abzubildenden Objekt definiert. Hierdurch wird stets der besagte Abstand konstant gehalten und eine einfache Berechnung der Bewegung in Kugelkoordinaten ermöglicht. Zusätzlich kann auch der Strahlendetektor auf der Kugeloberfläche oder alternativ auf einer Kugeloberfläche einer Kugel mit kleinerem oder größerem Radius bewegt werden.

Die erste Bewegung wird typischerweise mit einer niedrigeren Frequenz als die zweite Bewegung durchgeführt, jedoch können auch beide Bewegungen mit der gleichen Frequenz oder die erste Bewegung mit einer höheren Frequenz als die zweite Bewegung durchgeführt werden. Durch eine Wahl der Frequenz kann eine Abtastung entsprechend den Anforderungen in einfacher Weise erreicht werden und somit Artefakte in den Abbildungen aufgrund der häufigeren Aufnahmen von Bereichen, die in bisherigen Aufnahmen abgedeckt waren, effizient reduziert werden. Alternativ oder zusätzlich können die erste Bahn und die zweite Bahn auch abschnittsweise verschieden voneinander sein.

Vorzugsweise weisen die Frequenzen der ersten Bewegung und der zweiten Bewegung ein bestimmtes Verhältnis zueinander auf. Typischerweise beträgt dieses Verhältnis mindestens oder genau 1:2, 1:3 oder 1:4, d. h. die zweite Bewegung verläuft gerade mit der doppelten, dreifachen oder vierfachen Frequenz als die zweite Bewegung, um möglichst viele verschiedene Projektionsperspektiven zu erhalten. Alternativ ist es mit den zuvor genannten Bedingungen möglich, dass die erste Bewegung eine höhere Frequenz als die zweite Bewegung aufweist.

Außerdem kann die Auswerteeinheit eingerichtet sein, sämtliche Projektionsabbildungen zu einer dreidimensionalen Projektionsabbildung zu verarbeiten und hierbei durch Artefakte abgeschattete Gebiete automatisch durch eine oder mehrere Aufnahmen des abgeschatteten Gebiets unter einem anderen Aufnahmewinkel zu korrigieren. Ferner umfasst die Auswerteeinheit vorzugsweise eine Ausgabeeinheit, auf der sowohl die aufgenommenen einzelnen Bilder als auch alternativ oder zusätzlich die rekonstruierte dreidimensionale Abbildung wiedergegeben werden kann. Die Auswerteeinheit ist typischerweise auch dazu eingerichtet, die Vorrichtung zu steuern.

Eine Vorrichtung zum Erzeugen einer dreidimensionalen Abbildung eines Objekts umfasst eine auf einer ersten, in einer ersten Ebene liegenden Bahn in einer ersten Bewegung um das abzubildende Objekt geführte Strahlenquelle, einen Strahlendetektor und eine Auswerteeinheit. Die Strahlenquelle ist zum Reduzieren von einem durch eine Abschottung erzeugten Artefakt der erhaltenden Abbildung zeitgleich zu der ersten Bewegung durch eine zweite Bewegung auf einer zweiten Bahn relativ zum abzubildenden Objekt bewegbar. Die erste Bewegung und die zweite Bewegung überlagern sich hierbei und ein Abstand zwischen der Strahlenquelle und dem abzubildenden Objekt während des Überlagerns der ersten Bewegung und der zweiten Bewegung ist konstant. Somit können aus verschiedenen Perspektiven mehrere Aufnahmen des abzubildenden Objektes gemacht werden, sodass durch die zusätzlichen Projektionsperspektiven zusätzliche Bildinformationen erhalten werden. Vorzugsweise verläuft hierbei sowohl die erste Ebene als auch die zweite Ebene durch das abzubildende Objekt.

Die Vorrichtung ist typischerweise zum Durchführen des bereits beschriebenen Verfahrens geeignet. Es kann ferner vorgesehen sein, dass der Strahlendetektor bewegbar ist, wobei der Strahlendetektor vorzugsweise auf der ersten Bahn und bzw. oder der zweiten Bahn bewegbar ist und bzw. oder die Strahlenquelle und bzw. oder der Strahlendetektor zum Durchführen der zweiten Bewegung gegenüber der Ebene der ersten Bahn bzw. der ersten Bewegung neigbar sind. Hierdurch wird eine Variabilität der Vorrichtung erhöht, in dem auch der Strahlendetektor zumindest eine der Bewegungen der Strahlenquelle mit vollzieht. Vorzugsweise sind die Strahlenquelle und der Strahlendetektor eingerichtet, sich stets einander gegenüberliegend zu befinden. Hierdurch wird sowohl während der ersten Bewegung als auch während der zweiten Bewegung eine klare geometrische Anordnung der Strahlenquelle und des Strahlendetektors zueinander ermöglicht, die eine Auswertung der erhaltenen Bildinformationen vereinfacht. Die Strahlenquelle und der Strahlendetektor können hierzu durch eine starre oder eine flexible, d.h. eine nicht starre Kopplung miteinander verbunden sein. Vorzugsweise ist dies durch einen Roboterarm als Kopplung verwirklicht, der über mehrere an dem Roboterarm angebrachte Achsen bewegt werden kann.

Die ersten Bahn und bzw. oder die zweite Bahn der Strahlenquelle und bzw. oder des Strahldetektors können durch eine Linearachse, eine Rotationsachse und bzw. oder einen motorisierten Gelenkarm einstellbar sein. Somit werden auch die erste Bewegung bzw. die zweite Bewegung entsprechend eingestellt. Unter einem Gelenkarm soll dabei ein Arm mit mindestens einem Gelenk verstanden werden, bei dem über ein Gelenk zumindest eine Drehung um eine Achse vollführt werden kann. Typischerweise können der Gelenkarm nur rotatorische und nicht translatorisch verstellt werden. Vorzugsweise sind die Achsen direkt an der Strahlenquelle bzw. an dem Strahlendetektor angeordnet, sodass ein Drehmoment bei Bewegung der Strahlenquelle bzw. Strahlendetektors minimiert wird und nicht weitere Teile der Vorrichtung mit bewegt werden müssen.

Typischerweise ist die Vorrichtung eine Röntgenvorrichtung mit einer Röntgenquelle als Strahlenquelle und einem Röntgendetektor als Strahlendetektor. Der Strahlendetektor kann einen Flachdetektor mit einer Szintillatorschicht umfassen. Die Artefakte werden in diesem Fall durch eine Abschattung von Röntgenstrahlung, die auch durch eine Totalabsorption der Röntgenstrahlung gegeben sein kann, erzeugt. Die Auswerteeinheit ist vorzugsweise durch einen Computer mit einem Display realisiert und kann eingerichtet sein, ein iteratives Rekonstruktionsverfahren und bzw. oder ein angepasstes Rückprojektionsverfahren zu verwenden.

Ein erfindungsgemäßes Computerprogrammprodukt enthält eine Befehlsfolge, die eine Vorrichtung zum Erzeugen einer dreidimensionalen Abbildung eines Objekts steuert. Die Vorrichtung umfasst eine auf einer ersten, in einer ersten Ebene liegenden Bahn in einer ersten Bewegung um das abzubildende Objekt geführte Strahlenquelle, einen Strahlendetektor und eine Auswerteeinheit. Das Computerprogrammprodukt steuert die Vorrichtung derart, dass die Strahlenquelle zur Bildaufnahme relativ zum abzubildenden Objekt auf der ersten Bahn in der ersten Bewegung in mehrere Positionen bewegt wird, in denen jeweils mindestens ein Bild aufgenommen wird, wobei aus den aufgenommenen Bildern durch die Auswerteeinheit die dreidimensionale Abbildung rekonstruiert wird und die Strahlenquelle zum Reduzieren von einem durch eine Abschattung erzeugten Artefakt in der Abbildung zeitgleich zu der ersten Bewegung eine zweite Bewegung auf einer zumindest abschnittsweise von der ersten Bahn verschiedenen zweiten Bahn relativ zum abzubildenden Objekt durchführt. Die erste Bewegung und die zweite Bewegung überlagern sich hierbei und ein Abstand zwischen der Strahlenquelle und dem abzubildenden Objekt bleibt konstant während des Überlagerns der ersten Bewegung und der zweiten Bewegung. Das Computerprogrammprodukt ist typischerweise eingerichtet, die zuvor bereits beschriebene Vorrichtung anzusteuern und bzw. oder das zuvor bereits beschriebene Verfahren durchzuführen.

Das Durchführen des Verfahrens und bzw. oder das Ansteuern der Vorrichtung durch das Computerprogrammprodukt erfolgt typischerweise, wenn das Computerprogrammprodukt auf einer Recheneinheit abläuft.

Vorzugsweise kann das Computerprogrammprodukt direkt in einen internen Speicher der Recheneinheit geladen werden oder ist in diesem bereits gespeichert und umfasst typischerweise Teile eines Programmcodes zum Durchführen des beschriebenen Verfahrens oder zum Ansteuern der beschriebenen Vorrichtung, wenn das Computerprogrammprodukt auf der Recheneinheit abläuft bzw. ausgeführt wird. Das Computerprogrammprodukt kann auf einem maschinenlesbaren Träger, vorzugsweise einem digitalen Speichermedium gespeichert sein. Das Computerprogrammprodukt kann auch ein Computerprogramm umfassen, das Softwaremittel zur Durchführung des beschriebenen Verfahrens und bzw. oder zum Ansteuern der beschriebenen Vorrichtung aufweist, wenn das Computerprogramm in einem Automatisierungssystem oder auf der Recheneinheit ausgeführt wird.

Die zuvor bereits beschriebene Vorrichtung, das bereits beschriebene Verfahren und bzw. oder das Computerprogrammprodukt können in medizinischen Anwendungen, vorzugsweise im Rahmen einer digitalen Volumentomographie bzw. einer durchleuchtungsbasierten dreidimensionalen Bildgebung, von Zahnmedizin, Mundchirurgie, Kieferchirurgie, Gesichtschirurgie, Hals- Nasen-Ohren- Heilkunde und bzw. oder zerstörungsfreier industrieller Bildgebung verwendet werden.

Ausführungsbeispiele der Erfindung sind in den Zeichnungen dargestellt und werden nachfolgend anhand der Figuren 1 - 11 erläutert.

Es zeigen:
- Fig. 1:: eine schematische seitliche Ansicht einer Vorrichtung zur digitalen Volumentomographie nach dem Stand der Technik;
- Fig. 2:: eine Vorrichtung zum Erzeugen einer dreidimensionalen Abbildung eines Objekts, bei dem sowohl eine Strahlenquelle als auch ein Strahlendetektor in einer ersten Bewegung und einer zweiten Bewegung geführt werden;
- Fig. 3:: eine Fig. 2 entsprechende Darstellung der Vorrichtung, bei der die Strahlenquelle und der Strahlendetektor jedoch nicht miteinander gekoppelt sind;
- Fig. 4:: eine Fig. 2 entsprechende Darstellung der Vorrichtung, bei der nur die Strahlenquelle die zweite Bewegung vollführt;
- Fig. 5:: eine seitliche Ansicht eines in zwei Freiheitsgraden bewegbaren Tomographen;
- Fig. 6:: eine seitliche Ansicht eines Tomographen, bei dem die Strahlenquelle mittels einer Linearachse und einer Rotationsachse bewegbar ist;
- Fig. 7:: eine seitliche Ansicht eines Tomographen, bei dem die Strahlenquelle an einem zweiachsigen Roboterarm befestigt ist;
- Fig. 8:: eine Fig. 7 entsprechende Darstellung eines Tomographen, bei dem sowohl die Strahlenquelle als auch der Strahlendetektor jeweils an einem zweiachsigen Roboterarm befestigt sind;
- Fig. 9:: eine seitliche Ansicht eines Tomographen, bei dem sowohl die Strahlenquelle als auch der Strahlendetektor durch die Linearachse und die Rotationsachse bewegbar sind,
- Fig. 10:: einen zeitlichen Verlauf verschiedener Arten einer zweiten Bewegung und
- Fig.11:: eine Fig. 5 entsprechende Ansicht einer weiteren Ausführungsform eines in zwei Freiheitsgraden bewegbaren Tomographen.

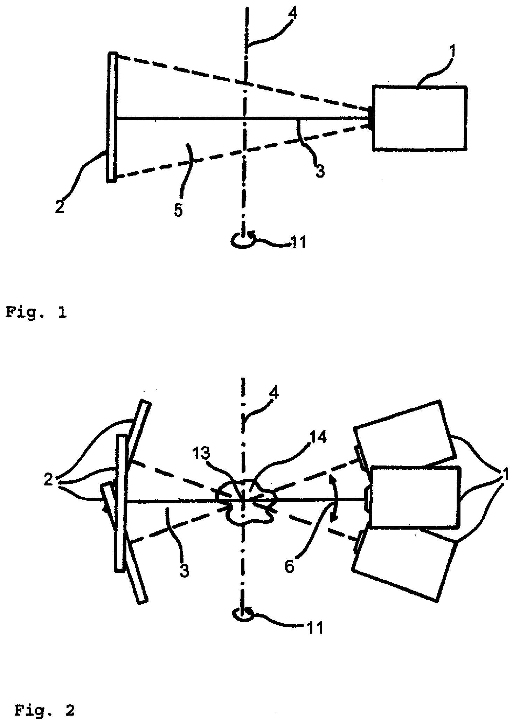

Fig. 1 zeigt in einer seitlichen Ansicht eine Vorrichtung zur digitalen Volumentomographie, also einen Volumentomographen, mit einer Röntgenquelle 1 und einem Röntgenflachdetektor 2 nach dem Stand der Technik. Die Röntgenquelle 1 und der Röntgenflachdetektor 2 sind einander gegenüberliegend angeordnet. Mittig durch die Röntgenquelle 1 und den Röntgenflachdetektor 2 verläuft eine Ebene einer Kreisbahn 3, auf der die Röntgenquelle 1 und der Röntgenflachdetektor 2 rotiert werden. Diese Rotation 11 erfolgt um die Rotationsachse 4, wobei sich die Röntgenquelle 1 und der Röntgenflachdetektor 2 während der Rotation 11 stets einander gegenüberliegend befinden. Die Röntgenquelle 1 sendet ein divergierend verlaufendes Bündel von Röntgenstrahlen 5 aus. Ein Mittelstrahl dieser Röntgenstrahlen 5 verläuft in der Ebene der Kreisbahn 3 und trifft mittig auf den Röntgenflachdetektor 2, der zumindest teilweise entlang des Strahlengangs positioniert ist. Die digitale Volumentomographie wird insbesondere in der Zahnmedizin, der Mundchirurgie, Kieferchirurgie und Gesichtschirurgie sowie der Hals-Nasen-Ohren-Heilkunde eingesetzt. Vornehmlich werden dreidimensionale Bilddaten der digitalen Volumentomographie zur diagnostischen Auswertung vom behandelnden Arzt verwendet.

Fig. 2 zeigt in einer seitlichen Ansicht eine erfindungsgemäße Vorrichtung, bei der die Röntgenquelle 1 und der Röntgenflachdetektor 2 im Gegensatz zu dem in Fig. 1 gezeigten Beispiel gleichzeitig und zusätzlich zu der Rotationbewegung 11 eine Neigungswinkelbewegung 6 vollführen können. Identische Merkmale sind in dieser wie auch in den folgenden Figuren mit jeweils gleichen Bezugszeichen gekennzeichnet. Die Röntgenquelle 1 und der Röntgenflachdetektor 2 sind derart miteinander gekoppelt, dass sie während der Rotationsbewegung 11 und der Neigungswinkelbewegung 6 stets einander gegenüber liegen. Die erste Bewegung um ein Zielvolumen 14 eines abzubildenden Objekts ist wiederum die Rotationsbewegung 11. Zusätzlich wird durch die Neigungswinkelbewegung 6 die Röntgenquelle 1 und der Röntgenflachdetektor 2 gegenüber der Ebene der Kreisbahn 3 erhöht oder abgesenkt.

Die Ebene der Kreisbahn 3 unterteilt somit einen den Tomographen umgebenden Raum in zwei Halbräume, wobei die Röntgenquelle 1 und der Röntgenflachdetektor 2 periodisch in einem der beiden Halbräume geführt werden. Die Ebene der Kreisbahn 3 liegt horizontal, sodass beispielsweise mit der in Figur 2 dargestellten Anordnung ein sitzender Patient untersucht werden kann. Somit befindet sich die Röntgenquelle 1 zeitweise oberhalb der horizontal liegenden Ebene der Kreisbahn 3. In diesen Positionen der Röntgenquelle 1 befindet sich der Röntgenflachdetektor 2 entsprechend unterhalb besagter Ebene. Sofern die Röntgenquelle 1 sich unterhalb der Ebene der Kreisbahn 3 befindet, durchläuft der Röntgenflachdetektor 2 oberhalb dieser Ebene gelegene Positionen. Falls die Ebene vertikal verläuft, würde sich die Anordnung aus der Röntgenquelle 1 und dem Röntgenflachdetektor 2 abschnittsweise links und rechts dieser Ebene statt oberhalb und unterhalb befinden. Anstelle des Röntgenflachdetektors 2 können in weiteren Ausführungsbeispielen auch andere Bauformen von Röntgendetektoren verwendet werden. Statt Röntgenstrahlung können natürlich auch andere Strahlenarten zusätzlich oder alternativ verwendet werden, solange sich diese als Durchleuchtungsstrahlung eignen.

Durch die Überlagerung der kreisförmigen Rotationsbewegung 11 mit der Neigungswinkelbewegung 6 mit mindestens einem weiteren mechanischen Freiheitsgrad werden Projektionsperspektiven des Zielvolumens 14 möglich, die mit einer Vorrichtung entsprechend Fig. 1 nicht zugänglich wären. Die Kreisbahn 3 ist bei dem in Fig. 2 dargestellten Ausführungsbeispiel eine geschlossene Kreisbahn, d.h. sowohl die Röntgenquelle 1 als auch der Röntgenflachdetektor 2 werden um 360° um das Zielvolumen 14 herum bewegt, wobei Anfangspunkt und Endpunkt der Rotation zusammenfallen. In weiteren Ausführungsbeispielen kann die Kreisbewegung jedoch auch nur einen bestimmten Winkelbereich, beispielsweise lediglich 180° umfassen. Ebenso kann in weiteren Ausführungsbeispielen die Röntgenquelle 1 und der Röntgenflachdetektor 2 statt auf einer Kreisbahn auf einer ellipsenförmigen Bahn oder einer Bahn mit frei wählbarem Verlauf geführt werden. Durch das beschriebene Verfahren können durch eine, zur kreisförmigen Bewegung übergeordnete Bewegung der Konfiguration aus Röntgenquelle 1 und Röntgenflachdetektor 2 Bildartefakte einer Bildgebung reduziert werden. Die Bahn der Neigungswinkelbewegung 6 schneidet die Kreisbahn 3 in regelmäßigen Abständen, sodass beide Bahnen abschnittsweise identisch zueinander und abschnittsweise verschieden voneinander sind. Eine resultierende Bahn aus der Bahn der Neigungswinkelbewegung 6 und der Kreisbahn 3 ergibt sich somit aus allen Raumpunkten, die die Röntgenquelle 1 bzw. der Röntgenflachdetektor 2 in zeitlicher Abfolge durchlaufen. Durch das Aufnehmen von Bildern aus unterschiedlichen Positionen, die durch die Neigebewegung 6 als zweiter Bewegung erreicht werden können, wird eine Auswertung aus mehreren Perspektiven ermöglicht.

In dem in Fig. 2 dargestellten Ausführungsbeispiel wird eine Umsetzung und eine Nutzung mindestens einer weiteren Bildaufnahmeebene, die durch ein abzubildendes Objekt verläuft, durch die Neigung der Konfiguration aus der Röntgenquelle 1 und dem Röntgenflachdetektor 2 gegenüber einem Zielvolumenzentrum angestrebt, um somit in den Bildaufnahmen auch eine Neigung des abzubildenden Objekts zu erreichen. Hierdurch werden zusätzliche Projektionsperspektiven erzeugt. Ein Abstand zwischen der Röntgenquelle 1 und dem Zielvolumen 14, der von einem dem abzubildenden Objekt am nächsten liegenden Punkt der zugewandten Oberfläche der Röntgenquelle 1 zu dem Mittelpunkt des Zielvolumens 14 entlang eines Zentralstrahls gemessen wird, ist während der Bewegung der Röntgenquelle 1 konstant. Ebenso ist ein in gleicher Weise bestimmter Abstand zwischen dem Röntgenflachdetektor 2 und dem Zielvolumen 14 konstant. In weiteren Ausführungsbeispielen können als Bezugspunkte für die Bestimmung der Abstände auch weitere Punkte verwendet werden, beispielsweise geometrische Mittelpunkt der Röntgenquelle 1, des Röntgenflachdetektors 2 oder des Zielvolumens 14 oder deren Masseschwerpunkte. Ebenso können als Bezugspunkte auch Punkte auf der Oberfläche der Röntgenquelle 1, des Röntgenflachdetektors 2 oder des Zielvolumens 14 verwendet werden.

Die Neigungswinkelbewegung 6 überstreicht in dem in Fig. 2 dargestellten Beispiel einen Winkelbereich von zwischen -45° bis 45° ausgehend von der Ebene der Kreisbahn 3, d. h. die Neigungswinkelbewegung 6 verläuft sowohl oberhalb als auch unterhalb besagter Ebene. In weiteren Ausführungsbeispielen kann natürlich auch vorgesehen sein, dass ein kleinerer oder ein größerer Winkelbereich bei der Neigungswinkelbewegung 6 durchfahren wird. Alternativ kann die Neigungswinkelbewegung 6 auch nur auf einer Seite der Ebene der Kreisbahn 3 erfolgen, d.h. beispielsweise nur oberhalb dieser Ebene oder nur unterhalb der Ebene. Auch die Neigungswinkelbewegung 6 ist in ihrem Verlauf frei wählbar. Sie kann beispielsweise sinusförmig oder in diskreten Schritten erfolgen. Typischerweise ist die Neigungswinkelbewegung 6 jedoch eine komplett periodische Bewegung, wobei eine Frequenz der Rotationsbewegung 11 gerade halb so groß wie eine Frequenz der Neigungswinkelbewegung 6 ist. Bei einem Umlauf der Rotationsbewegung erfolgen somit zwei Umläufe der Neigungswinkelbewegung. In weiteren Ausführungsbeispielen kann das Verhältnis der beiden Frequenzen zueinander natürlich auch andere Werte annehmen, beispielsweise 1:1 oder statt 1:2 auch 2:1. Somit erfolgt die Bewegung der Röntgenquelle 1 und des Röntgenflachdetektors 2 auf einer virtuellen Kugeloberfläche einer Kugel mit einem Radius, der gerade dem Abstand zwischen der Röntgenquelle 1 und dem Zielvolumens 14 bzw. zwischen dem Röntgenflachdetektor 2 und dem Zielvolumen 14 entspricht. Der Abstand zwischen der Röntgenquelle 1 und dem Zielvolumen 14 und der Abstand zwischen dem Röntgenflachdetektor 2 und dem Zielvolumen 14 kann dabei gleich groß, aber auch unterschiedlich groß sein. Typischerweise ist der Abstand zwischen der Röntgenquelle 1 und dem Zielvolumen 14 größer als der Abstand zwischen dem Röntgenflachdetektor 2 und dem Zielvolumen 14. Alternativ kann der Abstand zwischen der Röntgenquelle 1 und dem Zielvolumen 14 auch kleiner als der Abstand zwischen dem Röntgenflachdetektor 2 und dem Zielvolumen 14 sein.

Bei dem in Fig. 2 dargestellten Ausführungsbeispiel sind sowohl die Geschwindigkeit der ersten Bewegung, d.h. der Rotationsbewegung 11, und der zweiten Bewegung, d.h. der Neigungswinkelbewegung 6, konstant. Diese Geschwindigkeiten, d.h. die Winkelgeschwindigkeiten der Bewegung können in weiteren Ausführungsbeispielen natürlich auch zumindest abschnittsweise variabel sein. Hierbei kann eine Winkelgeschwindigkeit der zweiten Bewegung zumindest abschnittsweise höher als eine Winkelgeschwindigkeit der ersten Bewegung sein. In einem weiteren Ausführungsbeispiel wird bei einer Erhöhung der Winkelgeschwindigkeit der Neigebewegung als zweiter Bewegung eine Verringerung der Winkelgeschwindigkeit der Rotationsbewegung als der ersten Bewegung vorgenommen.

Zum Erzeugen einer dreidimensionalen Abbildung des Zielvolumens 14 des abzubildenden Objekts, im in Fig. 2 dargestellten Ausführungsbeispiel ein Gebiss als Zielvolumen 14 eines Schädels als das abzubildende Objekt, wird die Röntgenquelle 1 relativ zu dem Zielvolumen 14 sowie der Röntgenflachdetektor 2 durch die Kopplung mit der Röntgenquelle 1 in mehrere Positionen bewegt, in denen jeweils ein Bild aufgenommen wird. Statt genau eines Bildes in jeder Position aufzunehmen, kann natürlich auch eine Vielzahl von Bildern in dieser Position aufgenommen werden bzw. auch Positionen angefahren werden in denen zunächst keine Bilder aufgenommen werden. Aus den aufgenommenen Bildern wird durch eine in Fig. 2 nicht dargestellte Auswerteeinheit durch ein iteratives Volumenrekonstruktionsverfahren, wie einem Volumenrekonstruktionsalgorithmus, die dreidimensionale Abbildung des Zielvolumens 14 rekonstruiert. Alternativ oder zusätzlich kann auch ein angepasstes Rückprojektionsverfahren zum Berechnen der dreidimensionalen Abbildung des Zielvolumens verwendet werden. Die Auswerteeinheit umfasst einen Computer mit einer Ausgabeeinheit, beispielsweise einen Monitor. Auf der Ausgabeeinheit wird die dreidimensionale Abbildung des Zielvolumens 14 dargestellt, ebenso können auf dem Monitor auch zweidimensionale Bildaufnahmen, die in den angefahrenen Positionen gemacht wurden, ausgegeben werden. Eine Übermittlung von Bilddaten von dem Röntgenflachdetektor 2 zu der Auswerteeinheit kann sowohl durch ein Kabel als auch drahtlos erfolgen.

Ein Mittelpunkt - genauer ein Massenschwerpunkt - des abzubildenden Zielvolumens 14 liegt in einem Achsenschnittpunkt der Rotationsachse 4 mit der Neigungsachse 13. Statt des Massenschwerpunkts kann der Mittelpunkt allerding auch durch einen geometrischen Mittelpunkt resultierend aus Abmessungen des Zielvolumens 14 oder eine anatomisch auffällige und bzw. oder anderweitig interessante Region, die unabhängig von der Masse und der Geometrie des Objekts sein kann, gegeben sein. Während der Bewegung der Röntgenquelle 1 und des Röntgenflachdetektors 2 weist eine Oberflächennormale der genannten Geräte stets auf den Mittelpunkt des Zielvolumens 14 und ein Abstand zwischen der Röntgenquelle 1 und dem Mittelpunkt sowie ein Abstand zwischen dem Röntgenflachdetektor 2 bleibt konstant. In weiteren Ausführungsformen kann natürlich zumindest einer der beiden Abstände, aber auch beide Abstände während der Bewegungen variiert werden. Die Rotationsachse 4 steht senkrecht, d.h. im rechten Winkel auf der Ebene der Kreisbahn 3. Ebenso steht die Rotationsachse 4 senkrecht auf der Neigungsachse 13 und liegt typischerweise in einer Ebene der zweiten Bewegung. Die Neigungsachse 13 liegt komplett in der Ebene der Kreisbewegung 3.

Die Generierung der genannten zusätzlichen Projektionsperspektiven kann durch die in den folgenden Figuren anhand von verschiedenen Ausführungsbeispielen noch näher beschriebenen Systemansätze realisiert werden.

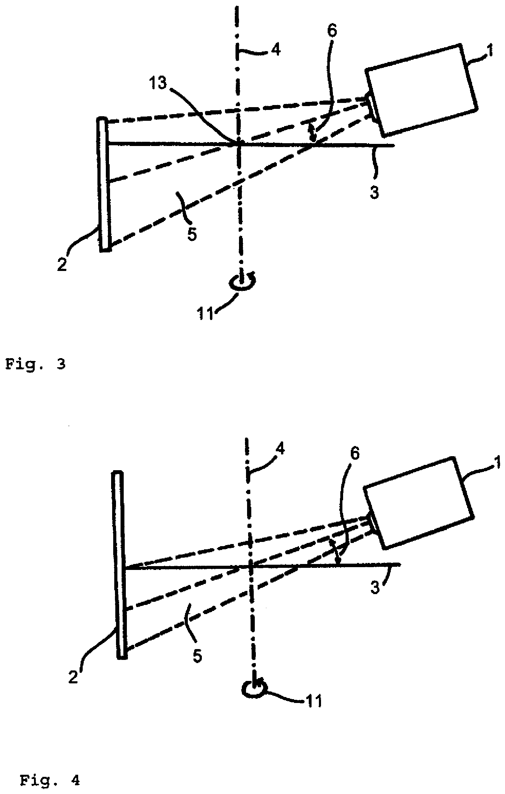

In Fig. 3 ist in einer Fig. 2 entsprechenden Darstellung ein Volumentomograph dargestellt, bei dem die Röntgenquelle 1 und der Röntgenflachdetektor 2 nicht mehr miteinander gekoppelt sind. Durch die fehlende Kopplung wird nun lediglich die Röntgenquelle 1 in der Rotationsbewegung 11 um die Rotationsachse 4 und die Neigungswinkelbewegung 6 um die Neigungsachse 13 geführt. Der Röntgenflachdetektor 2 - im dargestellten Ausführungsbeispiel ein Flachdetektor mit einer Szintillatorschicht - wird mit der Neigungswinkelbewegung 6 verlagert und vollführt die Rotationsbewegung 11 mit. Die von der Röntgenquelle 1 ausgesandte Röntgenstrahlung 5 trifft somit weitestgehend schräg, also nicht orthogonal im Gegensatz zu einem orthogonalen Auftreffen in den vorherigen Figuren, auf den Röntgenflachdetektor 2 auf. In weiteren Ausführungsformen kann jedoch auch vorgesehen sein, dass der Röntgenflachdetektor 2 zumindest die Neigungswinkelbewegung 6 ebenfalls vollführt, d. h. dass während der Rotationsbewegung 11, die sowohl die Röntgenquelle 1 als auch der Röntgenflachdetektor 2 vollführen, nicht nur die Röntgenquelle 1 durch Neigung verlagert wird. Der Röntgenflachdetektor 2 muss über die Rotationsbewegung 11 hinaus nicht verlagert werden, bewegt sich aber während der Neigungsbewegung 6 gegenläufig zur Röntgenquelle 1. Hierbei muss die Röntgenstrahlung nicht orthogonal auf den Röntgendetektor 2 auftreffen, dieser also nicht orthogonal zu dem Zentralstrahl stehen. Ebenso kann natürlich vorgesehen sein, dass der Röntgenflachdetektor 2 nur die Rotationsbewegung 11, jedoch nicht die Neigungswinkelbewegung 6 durchführt.

Fig. 4 zeigt in einer Fig. 2 entsprechenden Darstellung einen Volumentomographen, bei dem die Röntgenquelle 1 und der Röntgenflachdetektor 2 ebenfalls nicht miteinander gekoppelt sind. Der Röntgenflachdetektor 2 ist nun jedoch entsprechend vergrößert, um auch sämtliche von der Röntgenquelle 1 ausgesandten Röntgenstrahlen 5 durch die vergrößerte Detektorfläche auffangen zu können. Der Röntgenflachdetektor 2 und die Röntgenquelle 1 vollführen sowohl die erste Bewegung als auch die zweite Bewegung. Statt eines Flachdetektors 2 sind in weiteren Ausführungsformen natürlich auch Detektoren mit gewölbter Detektorfläche möglich.

Der in den bisherigen Figuren dargestellte digitale Volumentomograph kann mechanisch auf verschiedene Art und Weise realisiert werden.

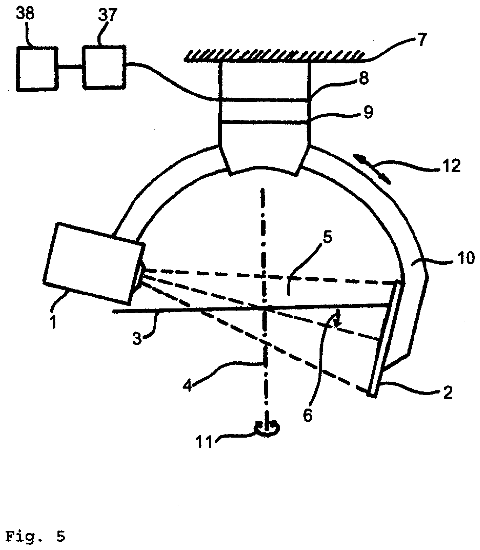

Fig. 5 zeigt in einer seitlichen Ansicht eine starre Verbindung zwischen der Röntgenquelle 1 und dem Röntgenflachdetektor 2 durch einen C-Bogen 10 als verbindendes Rahmenelement. Der in Fig. 5 dargestellte Volumentomograph ist an einer Decke 7 oder einem Gestell befestigt. An der Decke 7 ist eine angetriebene Drehachse 8 des Volumentomographen befestigt, die mit einer unterhalb der angetriebenen Drehachse 8 angebrachten angetriebenen Neigungsachse 9 in Verbindung steht. Die gesamte Anordnung ist mit der Auswerteeinheit 37 verbunden. Die erhaltenen Bilder, sowohl zweidimensional als auch dreidimensional, werden auf einem mit der Auswerteeinheit 37 verbundenen Monitor 38 ausgegeben.

Durch Drehung der angetriebenen Drehachse 8 ist sowohl die Neigungsachse 9 als auch der C-Bogen 10 rotierbar um die Rotationachse 4, die mittig durch die angetriebene Drehachse 8 und die angetriebene Neigungsachse 9 verläuft. Die angetriebene Neigungsachse 9 kann den C-Bogen 10 in einer Neigungsbewegung 12 führen, sodass die starr mit dem C-Bogen 10 verbundene Röntgenquelle 1 und der ebenso starr mit dem C-Bogen 10 verbundene Röntgenflachdetektor 2 die Neigungswinkelbewegung 6 durchführen, sodass der dargestellte Volumentomograph zwei Freiheitsgrade, nämlich rotierend und neigend, aufweist. Sämtliche der genannten Bewegungen können sowohl vollautomatisiert durch die angetriebene Drehachse 8 und die angetriebene Neigungsachse 9 als auch manuell einstellbar durchgeführt werden. Bei einer vollautomatisierten Durchführung ist auf dem Computer als der Auswerteeinheit 37 ein Computerprogrammprodukt gespeichert, welches den digitalen Volumentomograph entsprechend der in dem Computerprogrammprodukt enthaltenen Befehlsfolge ansteuert.

Die Bewegungen sind in dem in Fig. 5 dargestellten Ausführungsbeispiel automatisiert und können durch einen Benutzer in die Auswerteeinheit 37, die auch zum Steuern verwendet wird, einprogrammiert werden. Natürlich können die Positionen aber auch manuell von dem Benutzer angefahren werden.

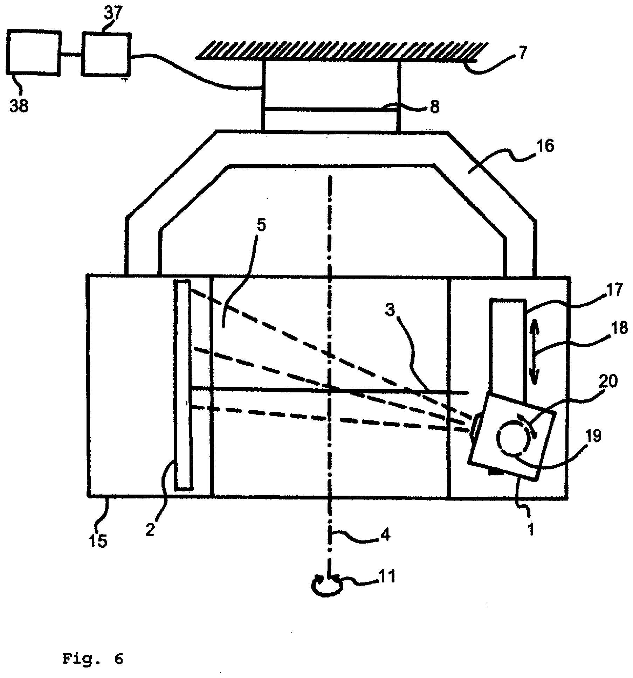

In Fig. 6 ist eine weitere seitliche Ansicht eines Ausführungsbeispiels des digitalen Volumentomographen dargestellt, bei dem die Neigungsbewegung 12 der Röntgenquelle 1 durch eine Linearachse 17 und eine Rotationsachse 19 erfolgt. Die angetriebene Drehachse 8 ist wiederum mit der Decke 7 verbunden, hält nun jedoch einen Befestigungsrahmen 16, der in weiteren Ausführungsformen auch ein außenliegendes Radiallager sein kann, an dessen Ende ein Gehäuse 15 befestigt ist. In dem Gehäuse 15 ist der Röntgenflachdetektor 2 gelagert. In weiteren Ausführungsformen kann auch ein Flachdetektor mit kleinerer Fläche verwendet werden, wobei dieser Flachdetektor auf einer Achse angeordnet ist, die den Flachdetektor aufwärts und abwärts bewegt. Ebenso ist in dem Gehäuse 15 dem Röntgenflachdetektor 2 gegenüberliegend die Linearachse 17 angeordnet, auf der die Röntgenquelle 1 in einer Linearbewegung 18 aufwärts und abwärts in vertikaler Richtung bewegt werden kann. Die Röntgenquelle 1 ist direkt auf der Linearachse 17 befestigt, kann jedoch zusätzlich über die Rotationsachse 19 in einer Drehbewegung 20 gedreht werden, sodass die Neigungswinkel 6 eine Bewegung um die Rotationsachse 19 realisiert wird.

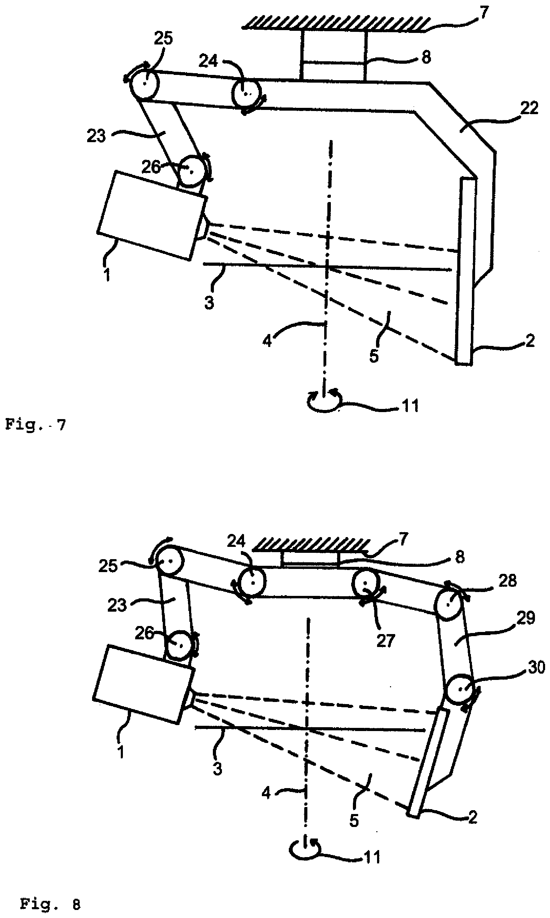

In Fig. 7 ist in einer seitlichen Ansicht eine weitere mögliche Realisierung des digitalen Volumentomographen dargestellt. An der angetriebenen Drehachse 8, die wiederum an der Decke 7 befestigt ist, ist ein Basisrahmen 22 angeordnet, mit dem der Röntgenflachdetektor 2 starr verbunden ist. Die Röntgenquelle 1 ist jedoch mit einem zweiachsigen Roboterarm 23 verbunden, der zwei Rotationsgelenke 24 und 25 aufweist. Über das Rotationsgelenk 24 ist der Roboterarm 23 mit dem Basisrahmen 22 verbunden. Die Röntgenquelle 1 ist über ein weiteres Rotationsgelenk 26 mit dem Roboterarm 23 verbunden. Das Rotationsgelenk 26 ist hierbei direkt an der Röntgenquelle 1 angeordnet, damit ein Drehmoment bei einer Bewegung der Röntgenquelle 1 möglichst minimal gehalten werden kann und keine weiteren Teile des Roboterarms 23 bewegt werden müssen. Ein Abstand zwischen dem Rotationsgelenk 24 und dem Rotationsgelenk 25 entspricht gerade einem Abstand zwischen dem Rotationsgelenk 25 und dem Rotationsgelenk 26. In weiteren Ausführungsformen kann dieser Abstand aber natürlich auch größer oder kleiner als beschrieben sein. Der mindestens zweiachsige Roboterarm 23 ermöglicht nur die Neigungsbewegung 12, während die Drehbewegung 20 übergeordnet bleibt durch Bewegen der kompletten Anordnung aus der Röntgenquelle 1, dem Röntgenflachdetektor 2, dem Basisrahmen 22 und dem Roboterarm 23.

Fig. 8 stellt eine Weiterentwicklung des in Figur 7 dargestellten digitalen Volumentomographen dar. Der Röntgenflachdetektor 2 ist nun ebenfalls an einem mindestens zweiachsigen Roboterarm 29 befestigt. Dieser Roboterarm 29 weist ebenfalls zwei Rotationsgelenke 27 und 28 auf. Der Röntgendetektor 2 ist mit dem Roboterarm 29 über ein Rotationsgelenk 30 verbunden, welches direkt an dem Röntgendetektor 2 anliegt. Die Rotationsgelenke 24, 25, 26, 27, 28 und 30 können jeweils um mindestens 300° rotiert werden. Ein Abstand von der Rotationsachse 4 zu dem Rotationsgelenk 24 entspricht gerade einem Abstand von der Rotationsachse 4 zu dem Rotationsgelenk 27. Generell sind der Roboterarm 23 und der Roboterarm 29 hinsichtlich der Anordnung ihrer Rotationsgelenke gleich aufgebaut. In weiteren Ausführungsformen können der Roboterarm 23 und der Roboterarm 29 natürlich auch unterschiedlich aufgebaut sein.

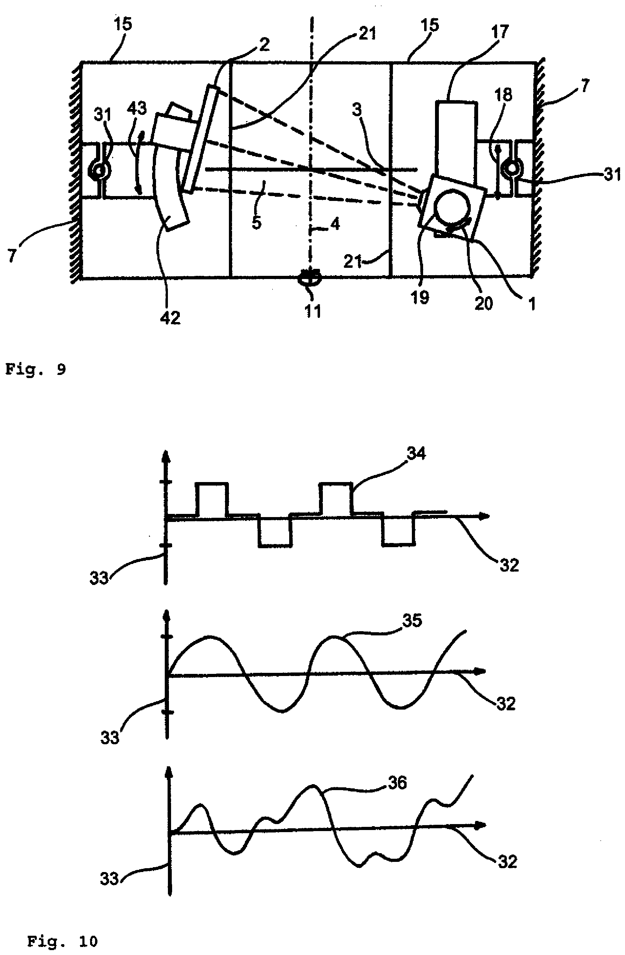

Fig. 9 stellt eine weitere Variante des digitalen Volumentomographen dar, bei der die Röntgenquelle 1 und der Röntgenflachdetektor 2 über ein Radiallager 31 und einen dazugehören Radialantrieb bewegt werden können. Die Röntgenquelle 1 ist hier wie in Figur 6 bereits dargestellt auf der Linearachse 17 und der Rotationsachse 19 bewegbar angeordnet. Die Linearachse 17 liegt jedoch auf dem Radiallager 31 auf und kann entlang diesem bewegt werden. Das Radiallager 31 ist mit einem Gestell 7 verbunden und an diesem befestigt. Ebenso ist der Röntgenflachdetektor 2 durch eine Bewegung 43 verschiebbar auf einer gekrümmten Linearachse 42 angeordnet. Die gekrümmte Linearachse 42 ist ebenfalls auf einem der Radiallager 31 gelagert und kann entsprechend auf diesem bewegt werden. Die Röntgenquelle 1, die Rotationsachse 19, die Linearachse 17 und das Radiallager 31 sind in dem Gehäuse 15 angeordnet. Die zwischen der Röntgenquelle 1 und dem Röntgenflachdetektor 2 liegenden Gehäuseteile 21 sind aus einem für Röntgenstrahlung durchlässigen Material. In weiteren Ausführungsformen kann auch das gesamte Gehäuse 15 aus einem für Röntgenstrahlung durchlässigen Material sein. Der Röntgenflachdetektor 2 ist ebenfalls innerhalb des Gehäuses 15 auf dem Radiallager 31 bewegbar angeordnet. Die in Fig. 9 dargestellte Ausführungsform kennzeichnet eine beidseitig offene Bauweise, mit der eine metallartefaktreduzierte digitale Volumentomographie für weite Bereiche eines menschlichen Körpers einsetzbar ist. Auch die in den Fign. 7-9 dargestellten Ausführungsbeispiele werden über die Auswerteeinheit 37 mit dem Monitor 38 angesteuert und erhaltene Bilddaten ausgewertet.

Die in den Fign. 5-9 dargestellten Anordnungen werden in der Medizin zur digitalen Volumentomographie verwendet, d.h. insbesondere bei zahnmedizinischen Untersuchungen oder in der Mundchirurgie, Kieferchirurgie, Gesichtschirurgie, bzw. Hals- Nasen- Ohren- Heilkunde.

In Fig. 10 sind drei mögliche zeitliche Verläufe der zweiten Bewegung dargestellt. Über einer Zeitachse 32 ist jeweils ein Neigungswinkelwert in Grad auf einer Neigungswinkelwertachse 33 aufgetragen. Auf der Zeitachse sind Zeiten in Sekunden aufgetragen. Der erste Verlauf 34 kennzeichnet einen diskreten Neigungswinkelverlauf, bei dem periodisch in diskreten Punkten der Neigungswinkel verändert wird. Bei dem zweiten dargestellten Verlauf 35 liegt ein harmonischer Verlauf des Neigungswinkels in einer Sinusform vor. Der dritte Verlauf 36 kennzeichnet schließlich einen komplett frei wählbaren Verlauf des Neigungswinkels, beispielsweise in Form eines Polynoms n-ten Grades, wobei "n" eine natürliche Grad kennzeichnet. Hierbei ist der Neigungswinkelbereich nicht konstant gehalten, sondern an eine Geometrie des abzubildenden Objekts angepasst. Im Allgemeinen bleibt die Winkelgeschwindigkeit der Rotationsbewegung 11 konstant und die konstante Rotationsbewegung 11 wird mit der Neigungswinkelbewegung 6 überlagert. Es ist allerdings in weiteren Ausführungsformen auch denkbar, die Winkelgeschwindigkeit der Rotationsbewegung 11 variabel halten. Dies ermöglicht eine schnelle Neigungswinkelbewegung 6 durch eine Anpassung der Winkelgeschwindigkeit der Rotationsbewegung 11 zu verlangsamen und somit eine mechanisch bedingte Genauigkeit durch verringerte Beschleunigungskräfte zu erhöhen.

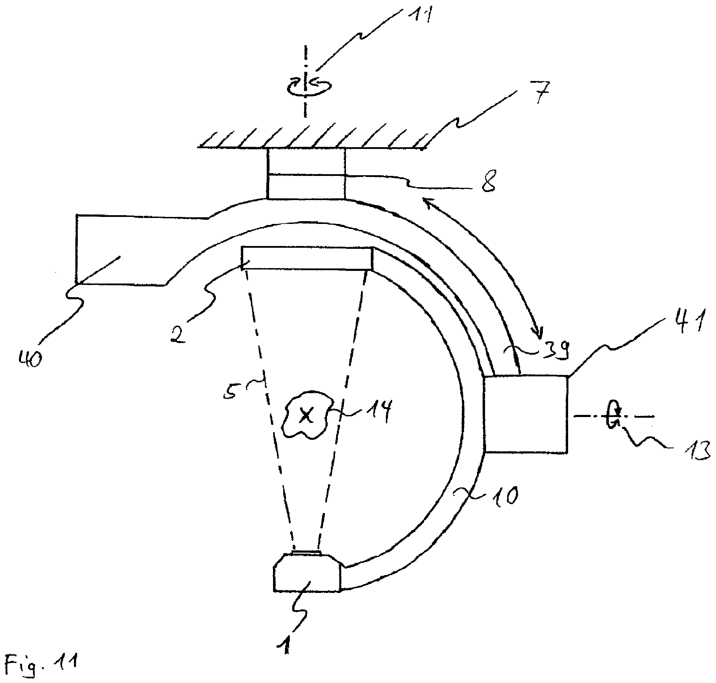

In Fig. 11 ist in einer Fig. 5 entsprechenden seitlichen Ansicht eine weitere Ausführungsform eines in zwei Freiheitsgraden bewegbaren Tomographen gezeigt. Der Tomograph ist wiederum an der Decke 7 oder einem Gestell mit der Drehachse 8 befestigt und kann in der Rotationsbewegung 11 um die Drehachse 8 gedreht werden. An der Drehachse 8 ist eine Führung 39 angebracht, die in Form eines Kreisbogens von 120° gehalten ist. An einem linken Ende der Führung 39 ist ein Gegengewicht bzw. ein Ausgleichsgewicht 40 angeordnet. Zwischen dem dem Ausgleichsgewicht 40 gegenüberliegenden Ende der Führung 39 und der Drehachse 8 ist ein Neigungsgelenk 41 führbar. In dem in Fig. 11 dargestellten Ausführungsbeispiel steht dieses Neigungsgelenk 41 in einem Winkel von 90° zu der Drehachse 8, jedoch können beliebige Winkel zwischen 0° und 90° eingestellt werden. An dem Neigungsgelenk 41 ist der C-Bogen 10 angeordnet, der um die Neigungsachse 13 gedreht werden kann. An einander gegenüberliegenden Enden des C-Bogens 10 sind der Röntgenflachdetektor 2 und die Röntgenquelle 1 angebracht, die um das Zielvolumen 14 gedreht und geneigt werden können.

### Bezugszeichenliste:

- 1: Röntgenquelle
- 2: Röntgenflachdetektor
- 3: Kreisbahn
- 4: Rotationsachse der Kreisbahn
- 5: Röntgenstrahlung
- 6: Neigungswinkelbewegung
- 7: Decke/Gestell
- 8: Drehachse
- 9: Neigungsachse
- 10: C-Bogen
- 11: Rotationsbewegung
- 12: Neigungsbewegung
- 13: Neigungsachse
- 14: Zielvolumen
- 15: Gehäuse
- 16: Befestigungsrahmen
- 17: Linearachse
- 18: Linearbewegung
- 19: Rotationsachse
- 20: Drehbewegung
- 21: röntgenstrahlungsdurchlässiges Gehäuse
- 22: Basisrahmen
- 23: Roboterarm
- 24: Rotationsgelenk
- 25: Rotationsgelenk
- 26: Rotationsgelenk
- 27: Rotationsgelenk
- 28: Rotationsgelenk
- 29: Roboterarm
- 30: Rotationsgelenk
- 31: Radiallager
- 32: Zeitachse
- 33: Neigungswinkelwertachse
- 34: diskreter Neigungswinkelverlauf
- 35: harmonischer Neigungswinkelverlauf
- 36: freier Neigungswinkelverlauf
- 37: Auswerteeinheit
- 38: Monitor
- 39: Führung
- 40: Ausgleichsgelenk
- 41: Neigungsgelenk
- 42: gekrümmte Linearachse
- 43: Bewegung

## Patentansprüche

1. Verfahren zum Erzeugen einer dreidimensionalen Abbildung eines Objekts (14) durch eine Abbildungsvorrichtung, die eine bewegliche Strahlenquelle (1), einen Strahlendetektor (2) und eine Auswerteeinheit (37) umfasst, wobei die Strahlenquelle (1) zur Bildaufnahme relativ zu dem abzubildenden Objekt (14) auf einer ersten, in einer ersten Ebene liegenden Bahn (3) in einer ersten Bewegung (11) in mehrere Positionen bewegt wird, in denen jeweils mindestens ein Bild aufgenommen wird, wobei aus den aufgenommenen Bildern durch die Auswerteeinheit (37) die dreidimensionale Abbildung rekonstruiert wird, und wobei die Strahlenquelle (1) zum Reduzieren von einem durch eine Abschattung erzeugten Artefakt in der Abbildung zeitgleich zu der ersten Bewegung (11) eine zweite Bewegung (6) auf einer zumindest abschnittsweise von der ersten Bahn verschiedenen zweiten Bahn relativ zu dem abzubildenden Objekt (14) durchführt, wobei sich die erste Bewegung (11) und die zweite Bewegung (6) überlagern, **dadurch gekennzeichnet, dass** die erste Bewegung (11) eine Kreisbewegung ist und ein Abstand zwischen der Strahlenquelle (1) und dem abzubildenden Objekt (14) während des Überlagerns der ersten Bewegung (11) und der zweiten Bewegung (6) konstant ist, so dass die Strahlenquelle (1) auf einer virtuellen Kugeloberfläche bewegt wird.

2. Verfahren nach Anspruch 1, **dadurch gekennzeichnet, dass** zumindest ein Teil des abzubildenden Objekts (14), vorzugsweise ein Mittelpunkt des abzubildenden Objekts, in einem Achsenschnittpunkt einer ersten Achse (4), um die die erste Bewegung (11) erfolgt, und einer zweiten Achse (13), um die die zweite Bewegung (6) erfolgt, liegt, wobei vorzugsweise die erste Achse (4) senkrecht auf der ersten Ebene steht.

3. Verfahren nach Anspruch 2, **dadurch gekennzeichnet, dass** die erste Achse (4) und die zweite Achse (13) senkrecht aufeinander stehen.

4. Verfahren nach einem der vorhergehenden Ansprüche, **dadurch gekennzeichnet, dass** die zweite Bewegung (6) zumindest abschnittsweise in einem von zwei durch die erste Ebene definierten Halbräumen erfolgt, wobei vorzugsweise die zweite Bewegung (6) zumindest abschnittsweise in beiden Halbräumen erfolgt und/oder die zweite Bewegung (6) zumindest abschnittsweise eine periodische Bewegung (35), vorzugsweise eine sinusförmig oder in diskreten Schritten (34) erfolgende Bewegung ist, oder in einem frei wählbaren Verlauf (36) erfolgt.

5. Verfahren nach einem der vorhergehenden Ansprüche, **dadurch gekennzeichnet, dass** die zweite Bewegung (6) eine Neigebewegung ist, wobei zumindest die Strahlenquelle in der Kreisbewegung auf einer Kreisbahn um mindestens 90°, vorzugsweise um mindestens 180°, besonders vorzugsweise um mindestens 360° bewegt wird und/oder zumindest die Strahlenquelle in der Neigebewegung gegenüber einer Ebene der Kreisbewegung um maximal zwischen 1° und 45°, vorzugsweise um maximal zwischen 10° und 35°, besonders vorzugsweise um maximal zwischen 15° und 30° geneigt ist.

6. Verfahren nach einem der vorhergehenden Ansprüche, **dadurch gekennzeichnet, dass** eine Geschwindigkeit der ersten Bewegung (11) und/oder eine Geschwindigkeit der zweiten Bewegung (6) konstant oder abschnittsweise variabel und/oder
ein Abstand zwischen der Strahlenquelle (1) und dem abzubildenden Objekt (14) kleiner oder gleich einem Abstand zwischen dem Strahlendetektor (2) und dem abzubildenden Objekt (14) ist.

7. Verfahren nach einem der vorhergehenden Ansprüche, **dadurch gekennzeichnet, dass** die Strahlenquelle (1) das abzubildende Objekt während des Aufnehmens vollständig umläuft und/oder
während des Aufnehmens nur die Strahlenquelle (1) die erste Bewegung und die zweite Bewegung vollführt und der Strahlendetektor (2) nur die erste Bewegung vollführt.

8. Verfahren nach einem der vorhergehenden Ansprüche, **dadurch gekennzeichnet, dass** die erste Bewegung und die zweite Bewegung periodische Bewegungen sind, wobei vorzugsweise die zweite Bewegung eine höhere Frequenz als die erste Bewegung aufweist und/oder eine Winkelgeschwindigkeit der zweiten Bewegung zumindest abschnittsweise höher als eine Winkelgeschwindigkeit der ersten Bewegung ist.

9. Verfahren nach einem der vorhergehenden Ansprüche, **dadurch gekennzeichnet, dass** bei einem Erhöhen der Winkelgeschwindigkeit der zweiten Bewegung ein Herabsetzen der Winkelgeschwindigkeit der ersten Bewegung erfolgt und/oder
die rekonstruierte dreidimensionale Abbildung und/oder die aufgenommenen Bilder von der Auswerteeinheit ausgegeben werden.

10. Vorrichtung zum Erzeugen einer dreidimensionalen Abbildung eines Objekts (14), umfassend eine auf einer ersten, in einer ersten Ebene liegenden Bahn (3) in einer ersten Bewegung (11) in mehreren Positionen, in denen jeweils mindestens ein Bild aufgenommen wird, um das abzubildende Objekt (14) geführte Strahlenquelle (1), einen Strahlendetektor (2) und eine Auswerteeinheit (37), wobei aus den aufgenommenen Bildern durch die Auswerteeinheit (37) die dreidimensionale Abbildung rekonstruiert wird, und wobei die Strahlenquelle (1) zum Reduzieren von einem durch eine Abschattung erzeugten Artefakt der erhaltenen Abbildung zeitgleich zu der ersten Bewegung (11) durch eine zweite Bewegung (6) auf einer zumindest abschnittsweise von der ersten Bahn verschiedenen zweiten Bahn relativ zu dem abzubildenden Objekt (14) bewegbar ist, wobei sich die erste Bewegung (11) und die zweite Bewegung (6) überlagern, **dadurch gekennzeichnet, dass** die erste Bewegung (11) eine Kreisbewegung ist und ein Abstand zwischen der Strahlenquelle (1) und dem abzubildenden Objekt (14) während des Überlagerns der ersten Bewegung (11) und der zweiten Bewegung (6) konstant ist, so dass sich die Strahlenquelle (1) auf einer virtuellen Kugeloberfläche bewegt.

11. Vorrichtung nach Anspruch 10, **dadurch gekennzeichnet, dass** der Strahlendetektor (2) bewegbar ist, wobei der Strahlendetektor (2) vorzugsweise auf der ersten Bahn (3) und/oder der zweiten Bahn bewegbar ist und/oder die Strahlenquelle (1) und/oder der Strahlendetektor (2) zum Durchführen der zweiten Bewegung gegenüber der Ebene der ersten Bewegung (11) neigbar sind, wobei vorzugsweise die Strahlenquelle (1) und der Strahlendetektor (2) eingerichtet sind, sich stets einander gegenüberliegend zu befinden.

12. Vorrichtung nach einem der Ansprüche 10 oder 11, **dadurch gekennzeichnet, dass** die erste Bahn (3) und/oder die zweite Bahn der Strahlenquelle (1) und/oder des Strahlendetektors (2) durch eine Linearachse (17), eine Rotationsachse (19) und/oder einen motorisierten Gelenkarm (23, 29) einstellbar ist, wobei der motorisierte Gelenkarm vorzugsweise ein Roboterarm ist, der mindestens drei Gelenke (Gelenke (24, 25, 26, 27, 28, 30) aufweist.

13. Vorrichtung nach einem der Ansprüche 10 bis 12, **dadurch gekennzeichnet, dass** die Vorrichtung eine Röntgenvorrichtung mit einer Röntgenquelle als Strahlenquelle (1) und einem Röntgendetektor als Strahlendetektor (2) ist.

14. Computerprogrammprodukt, enthaltend eine auf einem maschinenlesbaren Träger gespeicherte Befehlsfolge zum Durchführen des Verfahrens nach einem der Ansprüche 1-9 und/oder zum Ansteuern der Vorrichtung nach einem der Ansprüche 10-13, wenn das Computerprogrammprodukt auf einer Recheneinheit abläuft.

15. Verwendung einer Vorrichtung nach einem der Ansprüche 10-13 und/oder eines Computerprogrammprodukts nach Anspruch 14 bzw. Anwendung eines Verfahrens nach einem der Ansprüche 1-9 im Rahmen einer digitalen Volumentomographie, von Zahnmedizin, Mundchirurgie, Kieferchirurgie, Gesichtschirurgie, Hals-Nasen-Ohren-Heilkunde und/oder zerstörungsfreier industrieller Bildgebung.

## Claims

1. Method for producing a three-dimensional image of an object (14) by way of an imaging device which comprises a movable beam source (1), a beam detector (2) and an evaluation unit (37), wherein the beam source (1) for picture recording, is moved relative to the object (14) to be imaged, on a first path (3) lying in a first plane, in a first movement (11) into several positions, in which at least one picture is recorded in each case, wherein the three-dimensional image is reconstructed from the recorded pictures by way of the evaluation unit, and wherein for reducing an artefact in the image and which is produced by a shadowing, the beam source (1) simultaneously to the first movement (11) carries out a second movement (6) relative to the object (14) to be imaged, on a second path which at least in sections is different to the first path, wherein the first movement (11) and the second movement (6) superimpose, **characterized in that** the first movement (11) is a circular movement and a distance between the beam source (1) and the object (14) to be imaged during the superposition of the first movement (11) and the second movement (6) is constant, so that the beam source (1) is moved on a virtual spherical surface.

2. Method according to claim 1, **characterized in that** at least a part of the object (14) to be imaged, preferably a centre point of the object to be imaged, lies in an axis intersection point of a first axis (4), about which the first movement (11) is effected, and a second axis (13), about which the second movement (6) is effected, wherein preferably the first axis (4) is perpendicular to the first plane.

3. Method according to claim 2, **characterized in that** the first axis (4) and the second axis (13) are perpendicular to one another.

4. Method according to one of the preceding claims, **characterized in that** the second movement (6) at least in sections is effected in one of two half-spaces defined by the first plane, wherein preferably the second movement (6) at least in sections is effected in both half-spaces and/or the second movement (6) at least in sections is a periodic movement (35), preferably movement effected sinusoidally or in discrete steps (34), or is effected in a freely selectable course (36).

5. Method according to one of the preceding claims, **characterized in that** the second movement (6) is an inclination movement, wherein at least the beam source is moved in the circular movement on a circular path about at least 90°, preferably about at least 190°, particularly preferably about at least 360°, and/or at least the beam source in the inclination movement is inclined with respect to a plane of the circular movement by maximally between of 1° and 45°, preferably by maximally between 10° and 35°, particularly preferably by maximally between 15° and 30°.

6. Method according to one of the preceding claims, **characterized in that** a speed of the first movement (11) and/or a speed of the second movement (6) is constant or variable in sections and/or
a distance between the beam source (1) and the object (14) to be imaged is smaller than or equal to a distance between the beam detector (2) and the object (14) to be imaged.

7. Method according to one of the preceding claims, **characterized in that** the beam source (1) completely revolves around the object to be imaged during the recording and/or
the beam source (1) carries out the first movement and the second movement and the beam detector (2) carries out only the first movement, during the recording.

8. Method according to one of the preceding claims, **characterized in that** the first movement and the second movement are periodic movements, wherein preferably the second movement has a higher frequency than the first movement and/or an angular speed of the second movement at least in sections is greater than an angular speed of the first movement.

9. Method according to one of the preceding claims, **characterized in that** a reduction of the angular speed of the first movement is effected given an increase of the angular speed of the second movement and/or
the reconstructed three-dimensional image and/or the recorded pictures are outputted by the evaluation unit.

10. Device for producing a three-dimensional image of an object (14), comprising a beam source (1) which is led about the object (14) to be imaged, on a first path (3) lying in a first plane, in a first movement (11) into several position, in which at least one picture is recorded in each case, a beam detector (2) and an evaluation unit (37), wherein the three-dimensional image is reconstructed from the recorded image by way of the evaluation unit (37), and wherein for reducing an artefact in the obtained image and which is produced by a shadowing, the beam source (1) simultaneously to the first movement (11) can be moved by a second movement (6) relative to the object (14) to be imaged, on a second path which at least in sections is different to the first path, wherein the first movement (11) and the second movement (6) superimpose, **characterized in that** the first movement (11) is a circular movement and a distance between the beam source (1) and the object (14) to be imaged during the superposition of the first movement (11) and the second movement (6) is constant, so that the beam source (1) moves on a virtual spherical surface.

11. Device according to claim 10, **characterized in that** the beam detector (2) is movable, wherein the beam detector (2) is preferably movable on the first path (3) and/or the second path and/or the beam source (1) and/or the beam detector (2) can be inclined with respect to the plane of the first movement (11) for carrying out the second movement, wherein preferably the beam source (1) and the beam detector (2) are configured to always be located lying opposite one another.

12. Device according to one of claims 10 or 11, **characterized in that** the first path (3) and/or the second path (3) of the beam source (1) and/or the beam detector (2) are settable by way of a linear pivot (17), a rotation pivot (19) and/or a motorized joint arm (23, 29), wherein the motorised joint arm is preferably a robot arm which comprises at least three joints (24, 25, 26, 27, 28, 30).

13. Device according to any one of claims 10 to 12, **characterized in that** the device is an X-ray device with an X-ray source as a beam source (1) and with an X-ray detector as a beam detector (2).

14. Computer program product, containing a command sequence stored on a machine-readable carrier, for carrying out the method according to one of claims 1-9 and/or for activating the device according to one of claims 10-13, when the computer program product runs on a computation unit.

15. Use of a device according to one of claims 10-13 and/or a computer program product according to claim 14 or the application of a method according to one of claims 1-9 in the framework of digital volume tomography, for dental medicine, oral surgery, jaw surgery, facial surgery, ears-nose-and-throat medicine and/or destruction-free industrial imaging.

## Revendications

1. Procédé de génération d'une reproduction en trois dimensions d'un objet (14) par un dispositif de représentation qui comprend une source de rayonnement (1), un détecteur de rayonnement (2) et une unité d'exploitation (37), où, pour la prise d'images, la source de rayonnement (1) est déplacée par rapport à l'objet (1) à représenter sur une première piste (3) située dans un premier plan, dans un premier mouvement (11) dans plusieurs positions, dans lesquelles, chaque fois, au moins une image est prise, où la représentation en trois dimensions est reconstruite par l'unité d'exploitation (37) à partir des images prises, et où la source de rayonnement (1) exécute, pour la réduction d'un artefact dans la représentation généré par une ombre, simultanément au premier mouvement (11), un deuxième mouvement (6) sur une deuxième piste, différente au moins par endroits de la première piste, par rapport à l'objet (14) à représenter, où le premier mouvement (11) et le deuxième mouvement (6) se superposent, **caractérisé en ce que** le premier mouvement (11) est un mouvement circulaire et une distance entre la source de rayonnement (1) et l'objet (14) à représenter est constante pendant la superposition du premier mouvement (11) et du deuxième mouvement (6) de sorte que la source de rayonnement (1) est déplacée sur une surface sphérique virtuelle.

2. Procédé selon la revendication 1, **caractérisé en ce qu'**au moins une partie de l'objet (14) à représenter, de préférence, un point central de l'objet à représenter, se situe au niveau d'un point d'intersection axial d'un premier axe (4), autour duquel s'effectue le premier mouvement (11), et d'un deuxième axe (13), autour duquel s'effectue le deuxième mouvement (6), où, de préférence, le premier axe (4) est perpendiculaire au premier plan.

3. Procédé selon la revendication 2, **caractérisé en ce que** le premier axe (4) et le deuxième axe (13) sont perpendiculaires l'un par rapport à l'autre.

4. Procédé selon l'une des revendications précédentes, **caractérisé en ce que** le deuxième mouvement (6) s'effectue au moins partiellement dans l'un parmi deux demis espaces définis par le premier plan, où, de préférence, le deuxième mouvement (6) s'effectue au moins partiellement dans les deux demis espaces, et/ou le deuxième mouvement (6) est au moins partiellement un mouvement périodique (35), de préférence, un mouvement s'effectuant sous forme sinusoïdale ou de pas (34) discrets, ou s'effectue dans une évolution (36) pouvant être choisie librement.

5. Procédé selon l'une des revendications précédentes, **caractérisé en ce que** le deuxième mouvement (6) est un mouvement d'inclinaison, où au moins la source de rayonnement est déplacée dans le mouvement circulaire sur une piste circulaire à au moins 90 °, de préférence, à au moins 180 °, de manière particulièrement préférée, à au moins 360 °, et/ou au moins la source de rayonnement est inclinée dans le mouvement d'inclinaison par rapport à un plan du mouvement circulaire au maximum entre 1 ° et 45 °, de préférence, au maximum entre 10 ° et 35 °, de manière particulièrement préférée au maximum entre 15 ° et 30 °.

6. Procédé selon l'une des revendications précédentes, **caractérisé en ce qu'**une vitesse du premier mouvement (11), et/ou une vitesse du deuxième mouvement (6), est constante ou variable par endroits et/ou
une distance entre la source de rayonnement (1) et l'objet (14) à représenter est inférieure ou égale à une distance entre le détecteur de rayonnement (2) et l'objet (14) à représenter.

7. Procédé selon l'une des revendications précédentes, **caractérisé en ce que** la source de rayonnement (1) fait le tour complet de l'objet à représenter pendant la prise d'images, et/ou
pendant la prise d'images, seule la source de rayonnement (1) exécute le premier mouvement et le deuxième mouvement et le détecteur de rayonnement (2) exécute seulement le premier mouvement.

8. Procédé selon l'une des revendications précédentes, **caractérisé en ce que** les premier et deuxième mouvements sont des mouvements périodiques, où, de préférence, le deuxième mouvement présente une fréquence supérieure à celle du premier mouvement, et/ou une vitesse angulaire du deuxième mouvement est au moins par endroits supérieure à une vitesse angulaire du premier mouvement.

9. Procédé selon l'une des revendications précédentes, **caractérisé en ce que**, lors d'une augmentation de la vitesse angulaire du deuxième mouvement, il y a une diminution de la vitesse angulaire du premier mouvement, et/ou
la représentation en trois dimensions reconstruite, et/ou les images prises, sont délivrées par l'unité d'exploitation.

10. Dispositif pour générer une représentation en trois dimensions d'un objet (14), comprenant une source de rayonnement (1) déplacée sur une première piste (3) située dans un premier plan dans un premier mouvement (11) dans plusieurs positions, dans lesquelles chaque fois au moins une image est prise autour de l'objet (14) à représenter, un détecteur de rayonnement (2) et une unité d'exploitation (37), où la représentation en trois dimensions est reconstruite à partir des images prises par l'unité d'exploitation (37), et où la source de rayonnement (1) peut être déplacée par rapport à l'objet (14) à représenter de manière simultanée au premier mouvement (11) par un deuxième mouvement (6) sur une deuxième piste au moins partiellement différente de la première piste, pour la réduction d'un artefact de la représentation obtenue généré par une ombre, où le premier mouvement (11) et le deuxième mouvement (6) se superposent, **caractérisé en ce que** le premier mouvement (11) est un mouvement circulaire et une distance entre la source de rayonnement (1) et l'objet (14) à représenter est constante pendant la superposition du premier mouvement (11) et du deuxième mouvement (6), de sorte que la source de rayonnement (1) se déplace sur une surface sphérique virtuelle.

11. Dispositif selon la revendication 10, **caractérisé en ce que** le détecteur de rayonnement (2) peut se déplacer, où le détecteur de rayonnement (2) peut se déplacer de préférence sur la première piste (3) et/ou la deuxième piste, et/ou la source de rayonnement (1), et/ou le détecteur de rayonnement (2) sont inclinables par rapport au plan du premier mouvement (11) pour l'exécution du deuxième mouvement, où, de préférence, la source de rayonnement (1) et le détecteur de rayonnement (2) sont installés pour se trouver constamment opposés l'un par rapport à l'autre.

12. Dispositif selon l'une des revendications 10 ou 11, **caractérisé en ce que** la première piste (3) et/ou la deuxième piste de la source de rayonnement (1) et/ou du détecteur de rayonnement (2) peuvent être réglées par un axe linéaire (17), un axe de rotation (19) et/ou un bras d'articulation (23, 29) motorisé, où le bras d'articulation motorisé est de préférence un bras de robot qui présente au moins trois articulations (Articulations 24, 25, 26, 27, 28, 30).

13. Dispositif selon l'une des revendications 10 à 12, **caractérisé en ce que** le dispositif est un dispositif de radiographie X avec une source de rayons X en tant que source de rayonnement (1) et un détecteur de rayons X en tant que détecteur de rayonnement (2).

14. Produit de programme informatique contenant une suite de commandes mémorisées sur un support lisible par machine pour l'exécution du procédé selon l'une des revendications 1 à 9, et/ou pour le fonctionnement du dispositif selon l'une des revendications 10 à 13, lorsque le produit de programme informatique se déroule sur une unité de calcul.

15. Utilisation d'un dispositif selon l'une des revendications 10 à 13 et/ou d'un produit de programme informatique selon la revendication 14, respectivement, utilisation d'un procédé selon l'une des revendications 1 à 9 dans le cadre d'une tomographie volumique numérisée, de la médecine dentaire, de la chirurgie de la bouche, de la chirurgie maxillaire, de la chirurgie de la face, de l'oto-rhino-laryngologie et/ou d'une représentation d'imagerie industrielle non destructive.
